# EUROPEAN PATENT SPECIFICATION

(11) **EP 4 433 029 B1**
(45) Date of publication and mention of the grant of the patent: **18.02.2026**
(21) Application number: 22896229.6
(22) Date of filing: 10.11.2022
(51) Int. Cl.: A61K 9/12, A61K 38/57, A61K 47/18, A61P 37/00

(54) **INHALABLE RECOMBINANT PROTEIN POWDER FORMULATION FOR TREATING GENETIC AND AUTOIMMUNE DISORDERS**
INHALIERBARE REKOMBINANTE PROTEINPULVERFORMULIERUNG ZUR BEHANDLUNG VON GENETISCHEN UND AUTOIMMUNERKRANKUNGEN
FORMULATION DE POUDRE DE PROTÉINE RECOMBINANTE INHALABLE POUR LE TRAITEMENT DE TROUBLES GÉNÉTIQUES ET AUTO-IMMUNS

(30) Priority: 16.11.2021 SG 10202112736W
(43) Date of publication of application: 25.09.2024
(73) Proprietor: Agency for Science, Technology and Research, Singapore 138632 (SG); National University Hospital (Singapore) Pte Ltd, Singapore 119228 (SG)
(72) Inventor: HENG, Wen Chien, Desmond, Singapore 138668 (SG); NG, Say Kong, Singapore 138668 (SG); ZHANG, Wei, Singapore 138668 (SG); LEE, Sie Huey, Singapore 627833 (SG); TEO, Woon Pei, Jeanette, Singapore 119228 (SG)
(74) Representative: HGF
(86) International application number: PCT/SG2022/050819
(87) International publication number: WO 2023/091081

(56) References cited:
- WO-A1-2021/083910
- WO-A2-2005/086915
- US-A1- 2009 117 193
- US-A1- 2021 085 764
- SONG S.: "Alpha-1 Antitrypsin Therapy for Autoimmune Disorders", CHRONIC OBSTR PULM DIS., vol. 5, no. 4, 5 October 2018 (2018-10-05), pages 289 - 301, XP055793172, [retrieved on 20230628], DOI: 10.15326/JCOPDF. 5.4.2018 .0131

## Description

### FIELD OF THE INVENTION

The present invention relates to an inhalable dry powder formulation for use in treating genetic and autoimmune disorders. In particular, the invention relates to an inhalable dry powder formulation comprising a recombinant alpha-1-antitrypsin protein and a force control agent comprising di-leucine and tri-leucine in combination thereof for use in treating genetic and autoimmune disorders.

### BACKGROUND

Alpha-1-antitrypsin (A1AT) deficiency is a genetic disorder that can result in serious lung diseases such as emphysema, chronic obstructive pulmonary disease (COPD) and bronchiectasis. A1AT has a protective function and in its absence, the enzymes of inflammatory cells, especially neutrophil elastase, have harmful elevated activity leading to lung damage. According to the World Health Organization (WHO) estimates, COPD is the third leading cause of death worldwide, causing about 3.23 million deaths in 2019. It is estimated that about 1-2 % of all COPD patients have A1AT deficiency, which translates to an afflicted worldwide population of around 0.65-1.3 million people. Approved therapy for these patients involves a lifetime of A1AT intravenous infusion that is derived and purified from donor plasma, and at a high dose of approximately 60 mg/kg/week. These plasma-derived products carry the risk of donor-derived virus contaminants, while intravenous delivery has the disadvantage of patient compliance (for example, pain, difficulty of administration, etc.) and cold chain distribution requirements.

It is therefore desirable to provide a formulation for use in treating genetic and autoimmune disorders that would reduce the risk of developing serious lung diseases or at least to provide an alternative.

### SUMMARY OF INVENTION

In accordance with a first aspect of this invention, an inhalable dry powder formulation is provided. The inhalable dry powder formulation comprises a recombinant alpha-1-antitrypsin protein; a force control agent comprising di-leucine and tri-leucine in combination thereof, wherein the force control agent is present in an amount of at least 50% by weight of the formulation; and pharmaceutically acceptable excipients including a sodium salt and a non-reducing sugar or a sugar alcohol or a combination thereof.

In some embodiments, the inhalable dry powder formulation is in crystalline form.

In accordance with a second aspect of this invention, an inhalable dry powder formulation for use in the treatment of genetic and autoimmune disorders is provided. The inhalable dry powder formulation comprises a recombinant alpha-1-antitrypsin protein; a force control agent comprising di-leucine and tri-leucine in combination thereof, wherein the force control agent is present in an amount of at least 50% by weight of the formulation; and pharmaceutically acceptable excipients including a sodium salt and a non-reducing sugar or a sugar alcohol or a combination thereof.

In accordance with a third aspect, the invention relates to the use of the dry powder formulation according to the present invention in the manufacture of a pharmaceutical composition for treating genetic and autoimmune disorders in a subject.

In accordance with a fourth aspect of this invention, a method for treating genetic and autoimmune disorders in a subject is provided. The method comprises administering to the subject an effective amount of a formulation comprising a recombinant alpha-1-antitrypsin protein; a force control agent comprising di-leucine and tri-leucine in combination thereof, wherein the force control agent is present in an amount of at least 50% by weight of the formulation; and pharmaceutically acceptable excipients including a sodium salt and a non-reducing sugar or a sugar alcohol or a combination thereof.

### BRIEF DESCRIPTION OF THE DRAWINGS

The above advantages and features of a formulation in accordance with this invention are described in the following detailed description and are shown in the drawings:
Figures 1(a) to (f) are Field Emission Scanning Electron Microscopy (FESEM) images of samples F1, F2 and F3 (top to bottom): Figures 1(a) to (c) are images of Fresh samples, while Figures (d) to (f) are samples that were stored for three months at 25°C and 40% RH.
Figures 2(a) to (c) are XRD patterns of fresh spray-dried alpha-1-antitrypsin (A1AT) formulations, stored at 25°C and 40% RH for 1 month and 3 months. (a) F1, (b) F2 and (c) F3.
Figures 3(a) to (c) illustrate the Dynamic Vapour Sorption (DVS) isotherm of (a) fresh F1 and after 1 month and 3 months' storage, (b) fresh F2 and after 1 month and 3 months' storage and (c) fresh F3 and after 1 month and 3 months storage at 25°C, 40% RH.
Figure 4 is a graph showing the *in-vitro* deposition of F1, F2 and F3 at 60 L/min. Stages 1 to 4 denote impactor stages, followed by their corresponding aerodynamic cut-off diameter in parentheses. The data is presented as mean ± SD (n=3).

### DETAILED DESCRIPTION

In the present invention, an inhalable dry powder formulation based on a recombinant alpha-1-antitrypsin (A1AT) protein has been developed. The inhalable dry powder formulation comprises a recombinant alpha-1-antitrypsin protein; a force control agent comprising di-leucine and tri-leucine in combination thereof, wherein the force control agent is present in an amount of at least 50% by weight of the formulation; and pharmaceutically acceptable excipients including a sodium salt and a non-reducing sugar or a sugar alcohol or a combination thereof.

In one embodiment, the formulation is in crystalline form. The crystalline form allows the formulation to be formulated into a solid dose configuration for use as an inhalable dry powder formulation. The crystalline form has improved physicochemical properties and it is less hygroscopic than amorphous form.

The crystalline form can be prepared using various methods known in the art. In an exemplary embodiment, the crystalline form is prepared by spray drying the formulation with the force control agent comprising di-leucine and tri-leucine in combination thereof to obtain the spray-dried crystalline particles. In this exemplary embodiment, the recombinant alpha-1-antitrypsin protein, the force control agent and the other excipients are mixed and then sprayed through a spray nozzle into a drying chamber. The eventual particles are subsequently collected as a crystalline powder via a cyclone. One skilled in the art will appreciate that other spray drying method can be used without departing from the invention. The spray-dried particles are stable, and they can be prepared into fixed solid dose that allows the formulation to be delivered directly to the lungs of an infected patient via an inhaler device. In some embodiments, a dry powder inhaler device is used, and the formulation is inhaled in powder aerosol form.

In the present invention, the recombinant alpha-1-antitrypsin (A1AT) protein is derived using non-plasma derived technology. In an exemplary embodiment, the recombinant alpha-1-antitrypsin protein is produced by a recombinant Chinese Hamster Ovary (CHO) cell line in a serum free culture medium using a fed-batch bioreactor process, purified then eluted out into a HEPES buffer (i.e. 30 mM HEPES and 300 mM NaCl).

In various embodiments, the force control agent comprises di-leucine and tri-leucine in a ratio ranging from 49:1 to 90:1, 9:1, 4:1, 7:3, 3:2 or 1:1. In some embodiments, the force control agent comprises di-leucine and tri-leucine in a ratio of 49:1, 9:1, 4:1, 7:3, 3:2 or 1:1. In a preferred embodiment, the force control agent comprises di-leucine and tri-leucine in a ratio of 4:1.

In various embodiments, the force control agent is present in the formulation in an amount of at least 50% by weight of the formulation. The use of the force control agent in the formulation provides several benefits including imparting hydrophobicity and crystallinity to the formulation, hence conferring moisture stability and an improved shelf-life to the powders. The force control agent also helps in facilitating powder aerodynamicity. The introduction of the amino acid during the particle formation process during spray drying induces particle surface corrugations, which in turn facilitates improved aerodynamic performance. Further, the force control agent also helps to increase hydrophobicity with the di-leucine-tri-leucine combination. The combination of di-leucine and tri-leucine as the force control agent allows stable crystalline powders to be formed.

In the present invention, excipients including a sodium salt and a non-reducing sugar or a sugar alcohol or a combination thereof are used. Each of the excipients can be present in an amount from 1 to 39% by weight of the formulation. The combination of excipients in this proportion confers excellent moisture protection and aerosol performance to the dry powder formulation.

As used herein, the non-reducing sugar includes raffinose, sucrose, trehalose, etc.

As used herein, the sugar alcohol includes mannitol, erythritol, sorbitol, maltitol, xylitol, lactitol, etc.

In various embodiments, the sodium salt is selected from the group consisting of sodium chloride, sodium sulfate, sodium acetate, sodium bicarbonate, sodium butyrate, sodium metabisulfite, sodium carbonate, sodium phosphate and sodium dihydrogen phosphate.

The inhalable dry powder formulation of the present invention has an increased bioactivity of up to 19% as compared to a pre-formulated recombinant alpha-1-antitrypsin protein (see Example 2 described hereinbelow). This unexpected increase in bioactivity is achieved by having the formulation in spray-dried powder form as compared to suspension form.

The inhalable dry powder formulation is developed for use in targeted A1AT therapy to the lungs. The formulation confers high aerosol performance, and it is prepared in the form of a direct lung-targeting solid dose that can be delivered directly to the lungs via the inhaler device. The direct lung-targeting solid-dose configuration improves efficacy of the formulation. The efficacious powders can be emitted by up to 60% to 71% using the inhaler device. This mode of delivery of the formulation allows non-intravenous route of administration of the formulation to the patient and thus, helps to improve patient compliance. The formulation is stable at room temperature and can be stored at room temperature for up to at least 3 months or longer, and this reduces the need for cold chain.

In a second aspect of this invention, an inhalable dry powder formulation for use in the treatment of genetic and autoimmune disorders is provided. The inhalable dry powder formulation comprises a recombinant alpha-1-antitrypsin protein; a force control agent comprising di-leucine and tri-leucine in combination thereof, wherein the force control agent is present in an amount of at least 50% by weight of the formulation; and pharmaceutically acceptable excipients including a sodium salt and a non-reducing sugar or a sugar alcohol or a combination thereof.

As used herein, "genetic disorders" refers to alpha-1 antitrypsin deficiency, cystic fibrosis, etc.

As used herein, "autoimmune disorders" refers to rheumatoid arthritis, panniculitis, vasculitis, rhinosinusitis, uveitis, pancreatitis, lupus erythematosus, diabetes, etc.

In a third aspect, the invention relates to the use of the dry powder formulation according to the present invention in the manufacture of a pharmaceutical composition for treating genetic and autoimmune disorders in a subject. The formulation comprises a recombinant alpha-1-antitrypsin protein; a force control agent comprising di-leucine and tri-leucine in combination thereof, wherein the force control agent is present in an amount of at least 50% by weight of the formulation; and pharmaceutically acceptable excipients including a sodium salt and a non-reducing sugar or a sugar alcohol or a combination thereof.

In a fourth aspect of this invention, a method for treating genetic and autoimmune disorders in a subject is provided. The method comprises administering to the subject an effective amount of a pharmaceutical composition comprising a recombinant alpha-1-antitrypsin protein, a force control agent comprising di-leucine and tri-leucine in combination thereof, wherein the force control agent is present in an amount of at least 50% by weight of the formulation; and pharmaceutically acceptable excipients including a sodium salt and a non-reducing sugar or a sugar alcohol or a combination thereof.

In various embodiments, the pharmaceutical composition is administered by inhalation of the pharmaceutical composition in aerosolized form. In one embodiment, the composition is administered by an inhaler device.

In yet another aspect, a delivery system comprising an inhaler device and an inhalable dry powder formulation of the present invention is provided.

The dry powder formulation and method of the present invention provide several advantages. One advantage is that the direct lung-targeting solid-dose configuration of the formulation not only improves efficacy of the formulation and enhances patient compliance, it also imparts stability to the formulation and thus, reduces the need for cold chain. The dry powder formulation has enhanced protein bioactivity when the recombinant alpha-1-antitrypsin protein is formulated into solid form with excipients. Contrary to existing methods used in the art, the non-plasma derived technology used in deriving the recombinant alpha-1-antitrypsin protein is safe, scalable and cheaper to deliver.

To facilitate a better understanding of the present invention, the following examples of specific embodiments are given. In no way should the following examples be read to limit or define the entire scope of the invention. One skilled in the art will recognize that the examples set out below are not an exhaustive list of the embodiments of this invention.

### EXAMPLES

### Example 1

In this Example, three formulations (F1, F2 and F3) comprising the compositions as shown in Table 1, with different type and amount of force control agent, were developed and tested.

**Table 1. Composition of A1AT dry powder formulations.**

| Ingredient | Amount (wt %) | | |
|---|---|---|---|
| | F1 | F2 | F3 |
| A1AT | 10 | 10 | 10 |
| NaCl | 20 | 20 | 20 |
| Trehalose | 20 | 20 | 20 |
| L-leucine | 50 | | |
| Di-leucine | | 50 | 40 |
| Tri-leucine | | | 10 |

Each of the formulations F1, F2 and F3 was obtained by spray drying the aqueous suspension containing recombinant A1AT (in HEPES buffer) at a variety of formulation conditions (Table 1) on a B-290 Mini Spray Dryer with a 0.7 mm diameter nozzle (Büchi Labortechnik AG, Flawil, Switzerland). The inlet temperature of the spray dryers and the total feed concentration of aqueous solutions were maintained at 120°C and 10 mg/ml, respectively. The other operating parameters used in this example were atomization rate of 742 L/h, aspiration rate of 35-57.6 m³/h and feed rate of 4 ml/min.

The crystalline form of the formulation was prepared by spray drying the formulation with a crystallinity-imparting force control agent comprising di-leucine and tri-leucine in combination to obtain the spray-dried crystalline particles. The recombinant protein, force control agent and other excipients were mixed and sprayed through a spray nozzle into a drying chamber. The eventual particles were then collected as a crystalline powder via a cyclone.

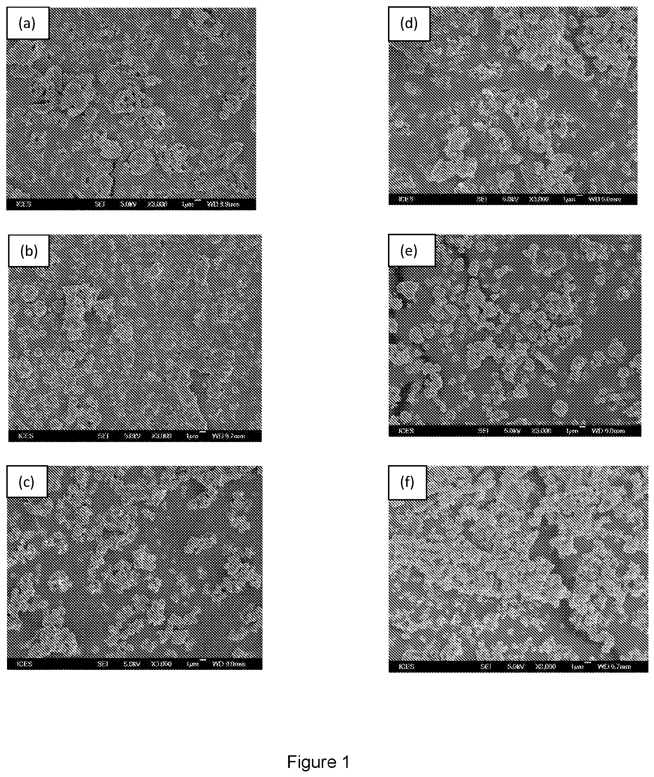

Figure 1 shows the morphology of the particles in all the three formulations. The figure shows that the morphology of the particles in all the three formulations were similar when compared between the freshly prepared and stored samples. The stored samples used in this example were stored for three months at 25°C and 40% relative humidity (RH). The results show that the samples were stable for the tested duration of 3 months, with no morphology change.

### Example 2

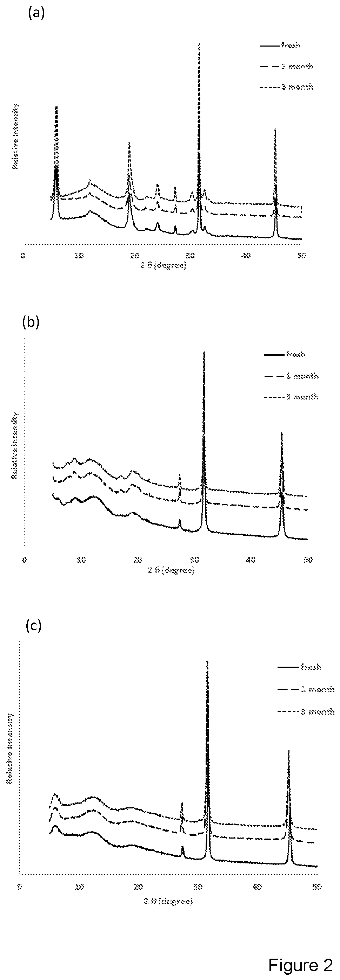

Several tests were carried out on the three formulations. The results of the tests were obtained and shown in the Figures 2 to 4.

### Crystalline form

The three formulations were prepared in crystalline form. Figure 2 shows that all three samples were crystalline and stable when stored, for at least up to three months of storage at 25°C and 40% RH.

### Moisture Sorption

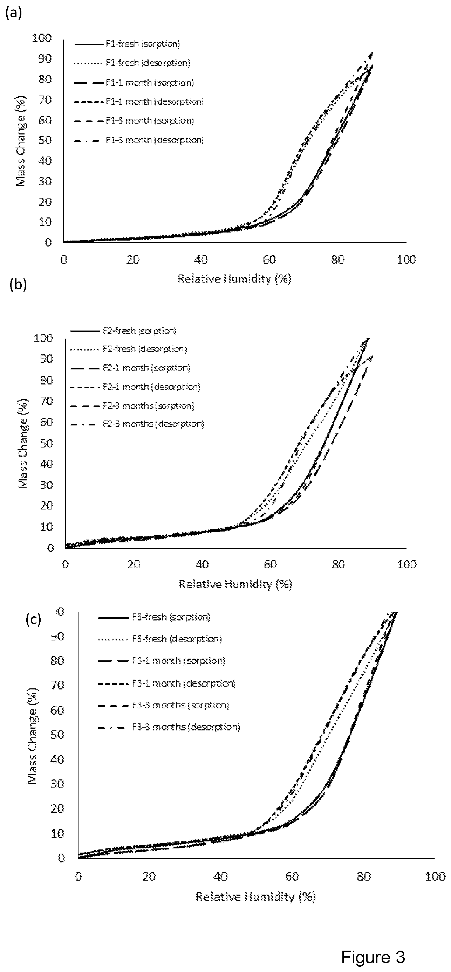

Figure 3 shows the results obtained from the Dynamic Vapour Sorption (DVS) isotherm tests carried on (a) fresh F1, and after 1 month and 3 months' storage; (b) fresh F2, and after 1 month and 3 months' storage; and (c) fresh F3, and after 1 month and 3 months' storage, all at 25°C and 40% RH.

The moisture sorption isotherms were obtained using the gravimetric vapour sorption (DVS Advantage 1, Surface Measurement Systems, UK) technique. Samples were weighed into a round-bottomed quartz sample pan and exposed to sorption-desorption cycles from 0 to 90% relative humidity (RH) at 25 °C and steps of 10% RH. Equilibrium moisture content at each humidity step was determined by a dm/dt of 0.02% per minute.

The results show that all three samples, F1, F2 and F3 exhibited reversible water sorption behaviour and were generally stable to moisture until about 60% RH. Of the three species, F3 was the most stable to moisture as there was a tighter overlap of cycles between the freshly prepared and stored samples.

### Aerosol Performance

The aerosol performance of the spray-dried powders was assessed using a multi-stage liquid impinger (MSLI, Copley Scientific, Nottingham, UK) coupled with a United State Pharmacopoeia (USP) stainless steel throat in a walk-in environmental chamber (Synersys, Singapore) maintained at 25°C and 40% RH. Prior to testing, 20 mL of deionized water was added across all four stages of the MSLI. Approximately 20 ± 2 mg of powder from each formulation was filled into a hydroxypropyl methylcellulose (HPMC) capsule (size 3, Capsugel^{®}, New Jersey, USA), loaded into the high efficiency Aerolizer^{®} inhaler (Novartis Pharmaceuticals, Basel, Switzerland) and pierced. The powder was then dispersed for 4s at 60 L/min. The flow through the MSLI was measured using a calibrated flow meter (TSI Model 4040C, TSI Instrument Ltd., Buckinghamshire, UK), controlled by a high capacity vacuum pump (Model HCP5, Copley Scientific, Nottingham, UK) and a critical flow controller (TPK 2000, Copley Scientific, Nottingham, UK). After dispersion, the device, capsule, throat and each stage of the MSLI were washed separately and thoroughly using deionized water. The solutions were then assayed by UV spectrophotometry (Agilent Technologies Cary 50 Conc, California, USA) at 280 nm after appropriate sample dilutions. In this study, fine particle fraction (FPF) represents the mass fraction of drug particles smaller than 5 µm in the aerosol cloud relative to the total mass recovered and was obtained by interpolation to the cumulative percent undersize at 5 µm. FPF(emitted) was obtained when the fine particle dose was expressed relative to the emitted dose. At a flow rate of 60 L/min, the aerodynamic cut-off diameters of stages 1, 2, 3 and 4 are 13.0, 6.8, 3.1 and 1.7 µm. Particles with diameter size less than 1.7 µm were captured on an integral filter paper.

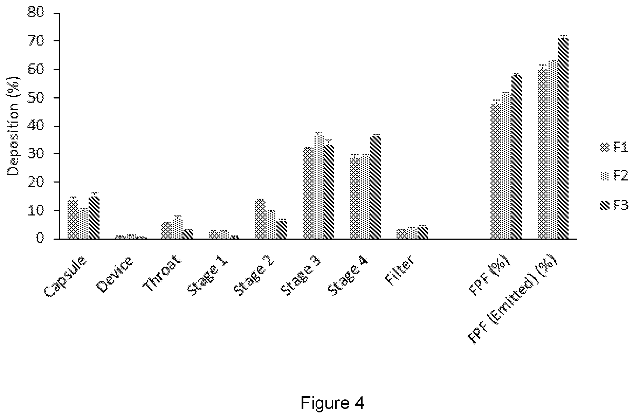

Figure 4 and Table 2 show that all three formulations are reasonably robust, that is, the. fine particle fraction (FPF) and FPF (emitted) is more than 30%, with F3 being the most aerodynamic (that is, FPF and FPF (emitted) of 57.9% and 71%, respectively). All the three formulations were stable when stored for three months at 25°C and 40% RH. See Table 2.

Figure 4 shows the *in-vitro* deposition of F1, F2 and F3 at 60 L/min. Stage 1, Stage 2, Stage 3 and Stage 4 denote impactor stages, followed by their corresponding aerodynamic cut-off diameter in parentheses. The data is presented as mean ± SD (n=3).

**Table 2. Deposition parameters of different formulations measured by MSLI at 60 L/min.**

| | FPF (%) | | | FPF (emitted) % | | |
|---|---|---|---|---|---|---|
| | F1 | F2 | F3 | F1 | F2 | F3 |
| Fresh | 47.8 ± 1.4 | 51.3 ± 0.5 | 57.9 ± 0.6 | 60.2 ± 1.3 | 62.9 ± 0.3 | 71.0 ± 0.9 |
| 1 month | 48.7 ± 3.2 | 52.9 ± 1.2 | 59.6 ± 0.6 | 61.2 ± L4 | 62.1 ± 1.5 | 70.6 ± 0.4 |
| 3 months | 51.0 ± 0.4 | 51.4 ± 0.2 | 60.7 ± 0.9 | 61.3 ± 0.5 | 62.1 ± 0.3 | 75.9 ± 0.4 |

### Bioactivity

The pre-formulated A1AT suspension (before spray drying) had a retained activity of 67.5 ± 4.9%. Interestingly, the freshly prepared formulated spray-dried powders had much improved activities of 73.4% to 80.9%. See Table 3 below. Protein solid formulation via excipients could be a useful approach to raising the activity profile of proteins. The powders were also generally stable when stored for three months at 25°C and 40% RH. We can see from Table 3 that F1 shows the highest activity preservation (at time = 0) among all the three formulations.

**Table 3. Bioactivity of fresh spray-dried A1AT formulations and after storage.**

| Retained Activity (%) | | | |
|---|---|---|---|
| | F1 | F2 | F3 |
| Fresh | 80.9 ± 2.0 | 79.4 ± 4.7 | 73.4 ± 1.5 |
| 1 month | 72.6 ± 9.4 | 75.4 ± 5.0 | 79.2 ± 4.7 |
| 3 months | 68.3 ± 8.0 | 76.6 ± 5.1 | 81.0 ± 10.5 |

### Results

From the results and tests carried out on the three formulations, F3 was the best performing formulation in terms of the fine particle fraction (FPF) and moisture stability, whereas F1 showed the highest activity preservation (at time = 0) among all species. Taken as a whole, a balance across all parameters and with due consideration to the shelf-life, the formulation that achieved most preferable results is F3.

## Claims

1. An inhalable dry powder formulation comprising:
a recombinant alpha-1-antitrypsin protein;
a force control agent comprising di-leucine and tri-leucine in combination thereof, wherein the force control agent is present in an amount of at least 50% by weight of the formulation; and
pharmaceutically acceptable excipients including a sodium salt and a non-reducing sugar or a sugar alcohol or a combination thereof.

2. The inhalable dry powder formulation according to claim 1, wherein the formulation is in crystalline form.

3. The inhalable dry powder formulation according to claim 1, wherein the di-leucine and the tri-leucine in the force control agent are present in a ratio ranging from 49:1 to 90:1, 9:1, 4:1, 7:3, 3:2 or 1:1.

4. The inhalable dry powder formulation according to claim 3, wherein the di-leucine and the tri-leucine in the force control agent are present in a ratio of 4:1.

5. The inhalable dry powder formulation according to any one of the preceding claims, wherein the formulation has a moisture content of less than 1% by weight based on the total weight of the formulation.

6. The inhalable dry powder formulation according to claim 1, wherein the inhalable dry powder formulation has an increased bioactivity of up to 19% as compared to a pre-formulated recombinant alpha-1-antitrypsin protein.

7. An inhalable dry powder formulation for use in the treatment of genetic and autoimmune disorders, the formulation comprising:
a recombinant alpha-1-antitrypsin protein;
a force control agent comprising di-leucine and tri-leucine in combination thereof, wherein the force control agent is present in an amount of at least 50% by weight of the formulation; and
pharmaceutically acceptable excipients including a sodium salt and a non-reducing sugar or a sugar alcohol or a combination thereof.

8. The inhalable dry powder formulation for use according to claim 7, wherein the formulation is in crystalline form.

9. The inhalable dry powder formulation for use according to any one of claims 7 to 8.
wherein the di-leucine and the tri-leucine in the force control agent are present in a ratio ranging from 49:1 to 90:1, 9:1, 4:1, 7:3, 3:2 or 1:1.

10. The inhalable dry powder formulation for use according to claim 9, wherein the di-leucine and the tri-leucine in the force control agent are present in a ratio of 4:1.

11. The inhalable dry powder formulation for use according to any one of claims 7 to 10.
wherein the formulation has a moisture content of less than 1% by weight based on the total weight of the formulation.

12. A delivery system comprising an inhaler device and an inhalable dry powder formulation as defined in claim 1.

## Patentansprüche

1. Inhalierbare Trockenpulverformulierung, umfassend:
ein rekombinantes Alpha-1-Antitrypsin-Protein;
ein Kraftsteuermittel, umfassend Dileucin und Trileucin in Kombination davon, wobei das Kraftsteuermittel in einer Menge von zumindest 50 Gew.-% der Formulierung vorliegt; und
pharmazeutisch unbedenkliche Hilfsstoffe, einschließlich eines Natriumsalzes und eines nichtreduzierenden Zuckers oder eines Zuckeralkohols oder einer Kombination davon.

2. Inhalierbare Trockenpulverformulierung gemäß Anspruch 1, wobei die Formulierung in kristalliner Form ist.

3. Inhalierbare Trockenpulverformulierung gemäß Anspruch 1, wobei das Dileucin und das Trileucin in dem Kraftsteuermittel in einem Verhältnis in einem Bereich von 49:1 bis 90:1, 9:1, 4:1, 7:3, 3:2 oder 1:1 vorliegen.

4. Inhalierbare Trockenpulverformulierung gemäß Anspruch 3, wobei das Dileucin und das Trileucin in dem Kraftsteuermittel in einem Verhältnis von 4:1 vorliegen.

5. Inhalierbare Trockenpulverformulierung gemäß einem der vorhergehenden Ansprüche, wobei die Formulierung einen Feuchtigkeitsgehalt von weniger als 1 Gew.-% basierend auf dem Gesamtgewicht der Formulierung aufweist.

6. Inhalierbare Trockenpulverformulierung gemäß Anspruch 1, wobei die inhalierbare Trockenpulverformulierung eine erhöhte Bioaktivität von bis zu 19 % im Vergleich zu einem vorformulierten rekombinanten Alpha-1-Antitrypsin-Protein aufweist.

7. Inhalierbare Trockenpulverformulierung zur Verwendung bei der Behandlung von genetischen und Autoimmunerkrankungen, die Formulierung umfassend:
ein rekombinantes Alpha-1-Antitrypsin-Protein;
ein Kraftsteuermittel, umfassend Dileucin und Trileucin in Kombination davon, wobei das Kraftsteuermittel in einer Menge von zumindest 50 Gew.-% der Formulierung vorliegt; und
pharmazeutisch unbedenkliche Hilfsstoffe, einschließlich eines Natriumsalzes und eines nichtreduzierenden Zuckers oder eines Zuckeralkohols oder einer Kombination davon.

8. Inhalierbare Trockenpulverformulierung zur Verwendung gemäß Anspruch 7, wobei die Formulierung in kristalliner Form ist.

9. Inhalierbare Trockenpulverformulierung zur Verwendung gemäß einem der Ansprüche 7 bis 8,
wobei das Dileucin und das Trileucin in dem Kraftsteuermittel in einem Verhältnis in einem Bereich von 49:1 bis 90:1, 9:1, 4:1, 7:3, 3:2 oder 1:1 vorliegen.

10. Inhalierbare Trockenpulverformulierung zur Verwendung gemäß Anspruch 9, wobei das Dileucin und das Trileucin in dem Kraftsteuermittel in einem Verhältnis von 4:1 vorliegen.

11. Inhalierbare Trockenpulverformulierung zur Verwendung gemäß einem der Ansprüche 7 bis 10,
wobei die Formulierung einen Feuchtigkeitsgehalt von weniger als 1 Gew.-% basierend auf dem Gesamtgewicht der Formulierung aufweist.

12. Abgabesystem, umfassend eine Inhalatorvorrichtung und eine inhalierbare Trockenpulverformulierung wie in Anspruch 1 definiert.

## Revendications

1. Formulation de poudre sèche inhalable comprenant :
une protéine alpha-1-antitrypsine recombinante ;
un agent de régulation de force comprenant de la di-leucine et de la tri-leucine en une combinaison de celles-ci, ledit agent de régulation de force étant présent en une quantité d'au moins 50 % en poids de la formulation ; et
des excipients pharmaceutiquement acceptables comprenant un sel de sodium et un sucre non réducteur ou un alcool de sucre ou une combinaison de ceux-ci.

2. Formulation de poudre sèche inhalable selon la revendication 1, ladite formulation se présentant sous forme cristalline.

3. Formulation de poudre sèche inhalable selon la revendication 1, ladite di-leucine et ladite tri-leucine dans l'agent de régulation de force étant présentes dans un rapport allant de 49:1 à 90:1, 9:1, 4:1, 7:3, 3:2 ou 1:1.

4. Formulation de poudre sèche inhalable selon la revendication 3, ladite di-leucine et ladite tri-leucine dans l'agent de régulation de force étant présentes dans un rapport de 4:1.

5. Formulation de poudre sèche inhalable selon l'une quelconque des revendications précédentes, ladite formulation comportant une teneur en humidité inférieure à 1 % en poids sur la base du poids total de la formulation.

6. Formulation de poudre sèche inhalable selon la revendication 1, ladite formulation de poudre sèche inhalable comportant une bioactivité accrue allant jusqu'à 19 % par rapport à une protéine alpha-1-antitrypsine recombinante préformulée.

7. Formulation de poudre sèche inhalable destinée à être utilisée dans le traitement de troubles génétiques et auto-immuns, la formulation comprenant :
une protéine alpha-1-antitrypsine recombinante ;
un agent de régulation de force comprenant de la di-leucine et de la tri-leucine en une combinaison de celles-ci, ledit agent de régulation de force étant présent en une quantité d'au moins 50 % en poids de la formulation ; et
des excipients pharmaceutiquement acceptables comprenant un sel de sodium et un sucre non réducteur ou un alcool de sucre ou une combinaison de ceux-ci.

8. Formulation de poudre sèche inhalable destinée à être utilisée selon la revendication 7, ladite formulation se présentant sous forme cristalline.

9. Formulation de poudre sèche inhalable destinée à être utilisée selon l'une quelconque des revendications 7 à 8, ladite di-leucine et ladite tri-leucine dans l'agent de régulation de force étant présentes dans un rapport allant de 49:1 à 90:1, 9:1, 4:1, 7:3, 3:2 ou 1:1.

10. Formulation de poudre sèche inhalable destinée à être utilisée selon la revendication 9, ladite di-leucine et ladite tri-leucine dans l'agent de régulation de force étant présentes dans un rapport de 4:1.

11. Formulation de poudre sèche inhalable destinée à être utilisée selon l'une quelconque des revendications 7 à 10, ladite formulation comportant une teneur en humidité inférieure à 1 % en poids sur la base du poids total de la formulation.

12. Système d'administration comprenant un dispositif inhalateur et une formulation de poudre sèche inhalable telle que définie dans la revendication 1.
